# DEMANDE DE BREVET EUROPEEN

(11) **EP 0 522 638 A1**
(43) Date de publication de la demande: **13.01.1993**
(21) Numéro de dépôt: 92201998.9
(22) Date de dépôt: 02.07.1992
(51) Int. Cl.: C07C 19/08, C07C 17/20

(54) **Procédé pour la préparation de 1,1-dichloro-1,3,3,3,-tétrafluoropropane**

(30) Priorité: 10.07.1991 BE 9100657
(71) Demandeur: SOLVAY (Société Anonyme), B-1050 Bruxelles (BE)
(72) Inventeur: Pennetreau, Pascal, B-1330 Rixensart (BE); Janssens, Francine, B-1800 Vilvoorde (BE); Barthelemy, Pierre, B-1370 Jodoigne (BE)
(74) Mandataire: Meyers, Liliane

(57) **Abrégé**

L'invention concerne un procédé pour la préparation de 1,1-dichloro-1,3,3,3-tétrafluoropropane par réaction en phase liquide de 1,1,1,3,3,3-hexachloropropane avec du fluorure d'hydrogène, en présence d'un catalyseur.

## Description

La présente invention concerne un procédé pour la préparation de 1,1-dichloro-1,3,3,3-tétrafluoropropane (HFA-234fb).

Elle concerne plus particulièrement un procédé de préparation de 1,1-dichloro-1,3,3,3-tétrafluoropropane au départ de 1,1,1,3,3,3-hexachloropropane, en une étape.

Suite au problème de l'appauvrissement de la couche d'ozone et aux limitations de production et d'utilisation imposées en conséquence pour les chlorofluorocarbures entièrement halogénés (CFC), il existe aujourd'hui un intérêt accru pour les hydrocarbures chlorofluorés partiellement halogénés (HFA). Parmi ceux-ci, le 1,1-dichloro-1,3,3,3-tétrafluoropropane pourrait notamment s'avérer être un substitut intéressant du 1,1,2-trichlro-1,2,2-trifluoroéthane (CFC-113).

Le certificat d'auteur SU 601912 de Shvarcman et al. a pour objet la préparation du 1,1-dichloro-1,3,3,3-tétrafluoropropane, qu'il décrit comme un composé de faible toxicité, utilisable comme narcotique et en tant qu'anesthésique par inhalation. Le 1,1-dichloro-1,3,3,3-tétrafluoropropane y est préparé par chloration photochimique de 1-chloro-3,3,3-trifluoropropane, suivie de la fluoration du 1,1,1-trichloro-3,3,3-trifluoropropane formé, par action de trifluorure d'antimoine (réactif) et en présence de pentachlorure d'antimoine (catalyseur).

Ce procédé met en oeuvre comme produit de départ un chlorotrifluoropropane, réactif déjà élaboré. Il nécessite ensuite une chloration et une fluoration. Pour cette dernière étape, le trifluorure d'antimoine est mis en oeuvre en quantités importantes.

La présente invention a pour but de procurer un procédé permettant la préparation de 1,1-dichloro-1,3,3,3-tétrafluoropropane, avec une bonne sélectivité, au départ de 1,1,1,3,3,3-hexachloropropane.

A cet effet, l'invention concerne un procédé pour la préparation de 1,1-dichloro-1,3,3,3-tétrafluoropropane par réaction en phase liquide de 1,1,1,3,3,3-hexachloropropane avec du fluorure d'hydrogène, en présence d'un catalyseur.

Le 1,1,1,3,3,3-hexachloropropane mis en oeuvre au départ du procédé selon l'invention s'obtient avantageusement par réaction du chlorure de vinylidène avec le tétrachlorométhane.

Il est ainsi possible d'obtenir le 1,1-dichloro-1,3,3,3-tétrafluoropropane en deux étapes, au départ de chlorure de vinylidène et de tétràchlorométhane, produits simples et largement disponibles.

Comme catalyseur de la réaction en phase liquide de 1,1,1,3,3,3-hexachloropropane avec du fluorure d'hydrogène, on peut mettre en oeuvre des catalyseurs d'hydrofluoration favorisant la substitution d'un atome de chlore par un atome de fluor. Parmi les catalyseurs utilisables, on peut citer les dérivés des métaux choisis parmi les métaux des groupes IIIa, IVa et b, Va et b, VIb du tableau périodique des éléments et leurs mélanges. On-retient plus spécialement les dérivés de titane, de tantale, de molybdène, de bore, d'étain et d'antimoine. De préférence, on met en oeuvre des dérivés d'étain ou d'antimoine. Les dérivés de l'étain conviennent particulièrement bien. Comme dérivés des métaux, on peut citer les sels et plus particulièrement les halogénures. De préférence, on choisit parmi les chlorures, les fluorures et les chlorofluorures. Des catalyseurs particulièrement préférés selon la présente invention sont les chlorures, les fluorures et les chlorofluorures d'étain et d'antimoine et leurs mélanges. Les chlorures conviennent particulièrement bien. Le tétrachlorure d'étain s'est révélé particulièrement intéressant, notamment en ce qu'il résulte de son utilisation une sélectivité élevée en 1,1-dichloro-1,3,3,3-tétrafluoropropane.

On peut également utiliser un cocatalyseur.

La quantité de catalyseur mise en oeuvre dans le procédé peut varier dans de larges limites. Elle est généralement d'au moins 0,005 mole de catalyseur par mole de 1,1,1,3,3,3-hexachloropropane. Le plus souvent, elle ne dépasse pas 0,1 mole de catalyseur par mole de 1,1,1,3,3,3-hexachloropropane. De préférence, elle est d'au moins 0,02 mole de catalyseur par mole de 1,1,1,3,3,3-hexachloropropane. Il est également préférable qu'elle n'excède pas 0,06 mole de catalyseur par mole de 1,1,1,3,3,3-hexachloropropane.

Le rapport molaire entre le fluorure d'hydrogène et le 1,1,1,3,3,3-hexachloropropane mis en oeuvre est généralement d'au moins 4. Le plus souvént, ce rapport molaire ne dépasse pas 20. De préférence, on travaille avec un rapport molaire d'au moins 6. Il est également préférable que ce rapport molaire ne dépasse pas 17. Une excellente sélectivité en 1,1-dichloro-1,3,3,3-tétrafluoropropane est obtenue avec un rapport molaire d'au moins 8.

La température à laquelle s'effectue la réaction selon le procédé est généralement d'au moins 50°C. Le plus souvent, elle ne dépasse pas 150°C. De préférence, elle est d'au moins 100°C. Il est également préférable qu'elle ne dépasse pas 120°C.

La pression est choisie de manière à maintenir le milieu réactionnel sous forme liquide. Elle varie en fonction de la température du milieu réactionnel. Elle est généralement d'au moins 2 bar. Le plus souvent, elle ne dépasse pas 50 bar. La pression est de préférence d'au moins 10 bar. Il est également préférable qu'elle ne dépasse pas 40 bar. Elle est plus préférentiellement encore d'au moins 20 bar. D'excellents résultats sont obtenus sans qu'il soit nécessaire de dépasser 30 bar.

La durée de la réaction nécessaire pour assurer une sélectivité optimale en 1,1-dichloro-1,3,3,3-tétrafluoropropane est variable en fonction des conditions opératoires et sera évaluée dans chaque cas par voie expérimentale. L'évolution de la composition du milieu réactionnel peut notamment être suivie par le dosage par chromatographie en phase vapeur de prélèvements réalisés à intervalles réguliers.

Le procédé selon l'invention peut être réalisé dans tout réacteur réalisé dans des matériaux résistant à la pression et au fluorure d'hydrogène. On utilise souvent des réacteurs réalisés en acier, en acier inoxydable ou en alliages tels que ceux connus sous les marques MONEL, INCONEL ou HASTELLOY. On peut également utiliser des réacteurs munis d'un revêtement en un métal ou alliage inerte, ou recouverts d'une couche d'une résine inerte dans les conditions de réaction, notamment des résines fluorées.

Comme déjà mentionné, le 1,1,1,3,3,3-hexachloropropane mis en oeuvre au départ du procédé selon l'invention peut être obtenu par télomérisation du chlorure de vinylidène avec le tétrachlorométhane et notamment de la manière décrite par Belbachir et al. (Makromol. Chem., 185, 1984, 1583-1595).

Cette réaction est avantageusement effectuée en présence d'un catalyseur du type des peroxydes ou des sels de fer ou de cuivre et, en particulier, du chlorure cuivreux, lequel offre une excellente sélectivité en 1,1,1,3,3,3-hexachloropropane produit.

Ladite réaction est par ailleurs avantageusement réalisée en présence d'un solvant polaire donneur tel que le diméthylsulfoxyde ou l'acétonitrile, l'acétonitrile offrant d'excellents résultats.

Avant sa mise en oeuvre dans le procédé selon l'invention, le 1,1,1,3,3,3-hexachloropropane peut être purifié, notamment par distillation sous pression réduite.

Les exemples qui suivent sont donnés dans le but d'illustrer l'invention mais ne sont nullement limitatifs.

### Exemple 1

### a. Préparation du 1,1,1,3,3,3-hexachloropropane

On utilise un autoclave de 1 l recouvert d'une résine fluorocarbonée TEFLON ^{R} , équipé d'un agitateur, d'une sonde de température et d'un tube plongeant permettant d'effectuer des prélèvements en cours d'essais.

On introduit dans cet autoclave 1,8 mole de chlorure de vinylidène, 3,6 mole de tétrachlorométhane, 0,018 mole de chlorure cuivreux et 3,6 mole d'acétonitrile.

Le milieu réactionnel est agité et chauffé à 140°C pendant 13 heures. La pression autogène atteint 5,9 bar.

On refroidit alors l'autoclave jusqu'à la température ambiante et on le vide.

L'analyse révèle que le taux de transformation du chlorure de vinylidène est alors de 95 % en moles et la sélectivité en 1,1,1,3,3,3-hexachloropropane est de 87 % molaires par rapport au chlorure de vinylidène transformé.

Le 1,1,1,3,3,3-hexachloropropane obtenu est purifié par distillation sous pression réduite.

### b. Hydrofluoration du 1,1,1,3,3,3-hexachloropropane

On utilise un autoclave de 0,5 l en acier inox, équipé d'un agitateur, d'un système de régulation de pression, d'une sonde de température et d'un túbe plongeant permettant d'effectuer des prélèvements en cours d'essais.

On fait le vide dans ce réacteur et on le refroidit à - 20°C.

On y introduit successivement 0,4 mole de 1,1,1,3,3,3-hexachloropropane, 0,02 mole de tétrachlorure d'étain et 3,6 mole de fluorure d'hydrogène.

Le milieu réactionnel est ensuite progressivement chauffé jusque 110°C et la pression est régulée à 25 bar.

Après 45 heures, le taux de transformation du 1,1,1,3,3,3-hexachloropropane est de 100 % et la sélectivité en 1,1-dichloro-1,3,3,3-tétrafluoropropane est de 50 % molaires par rapport au 1,1,1,3,3,3-hexachloropropane transformé. Après 115 heures, cette sélectivité atteint 79 %.

En fin de réaction, le milieu est refroidi et transvasé dans une ampoule à décanter en polyéthylène remplie d'eau, de manière à séparer le fluorure d'hydrogène n'ayant pas réagi. La phase organique est décantée et le 1,1-dichloro-1,3,3,3-tétrafluoropropane est purifié par distillation.

### Exemple 2

Le mode opératoire est analogue à celui de l'exemple 1.

On introduit successivement dans le réacteur 0,4 mole de 1,1,1,3,3,3-hexachloropropane, 0,02 mole de pentachlorure d'antimoine et 3,2 mole de fluorure d'hydrogène.

Le milieu réactionnel est ensuite progressivement chauffé jusque 110°C et la pression est régulée à 25 bar.

La réaction est très rapide.

Après 6 heures, le taux de transformation du 1,1,1,3,3,3-hexachloropropane est de 100 % et la sélectivité en 1,1-dichloro-1,3,3,3-tétrafluoropropane est de plus de 40 % molaires par rapport au 1,1,1,3,3,3-hexachloropropane transformé.

## Revendications

1. Procédé pour la préparation de 1,1-dichloro-1,3,3,3-tétrafluoropropane, caractérisé en ce que l'on fait réagir en phase liquide du 1,1,1,3,3,3-hexachloropropane avec du fluorure d'hydrogène, en présence d'un catalyseur.

2. Procédé selon la revendication 1, caractérisé en ce que le catalyseur est choisi parmi les dérivés des métaux des groupes IIIa, IVa et b, Va et b, VIb du tableau périodique des éléments et leurs mélanges.

3. Procédé selon la revendication 2, caractérisé en ce que les dérivés des métaux sont choisis parmi les chlorures, les fluorures et les chlorofluorures.

4. Procédé selon la revendication 1, caractérisé en ce que le catalyseur est choisi parmi les chlorures, les fluorures et les chlorofluorures d'étain et d'antimoine et leurs mélanges.

5. Procédé selon la revendication 4, caractérisé en ce que le catalyseur est le tétrachlorure d'étain.

6. Procédé selon la revendication 1, caractérisé en ce que le catalyseur est mis en oeuvre à raison de 0,005 à 0,1 mole de catalyseur par mole de 1,1,1,3,3,3-hexachloropropane.

7. Procédé selon la revendication 1, caractérisé en ce que le rapport molaire entre le fluorure d'hydrogène et le 1,1,1,3,3,3-hexachloropropane mis en oeuvre est d'au moins 4 et ne dépasse pas 20.

8. Procédé selon la revendication 1, caractérisé en ce que la température à laquelle la réaction est réalisée est d'au moins 50°C et ne dépasse pas 150°C.

9. Procédé selon la revendication 1, caractérisé en ce que la pression sous laquelle la réaction est réalisée est d'au moins 2 bar et ne dépasse pas 50 bar.

10. Procédé selon la revendication 1, caractérisé en ce que le 1,1,1,3,3,3-hexachloropropane mis en oeuvre est obtenu par réaction du chlorure de vinylidène avec le tétrachlorométhane en présence de chlorure cuivreux et d'acétonitrile.
